# EUROPEAN PATENT APPLICATION

(11) **EP 4 744 574 A1**
(43) Date of publication of application: **20.05.2026**
(21) Application number: 24213500.2
(22) Date of filing: 18.11.2024
(51) Int. Cl.: A61B 1/00, A61B 5/01

(54) **APPARATUS FOR AN OPTICAL IMAGING SYSTEM, OPTICAL IMAGING SYSTEM, METHOD AND COMPUTER PROGRAM**

(71) Applicant: Leica Instruments (Singapore) Pte. Ltd., Singapore 618299 (SG)
(72) Inventor: VAGEESAN, Naveen Kumar, 618299 Singapore (SG)
(74) Representative: 2SPL Patentanwälte PartG mbB

(57) **Abstract**

Examples relate to an apparatus for an optical imaging system comprising one or more processors and one or more storage devices. The apparatus may be configured to receive image data of a human tissue. The apparatus is configured to obtain thermal sensor data of a thermal sensor indicating a temperature of the human tissue. Further, the apparatus is configured to determine, based on the thermal sensor data, whether the temperature of the human tissue has exceeded a threshold value and/or to predict, based on the thermal sensor data, whether the temperature of the human tissue will exceed the threshold value. The apparatus is further configured to trigger an action if the temperature of the human tissue has exceeded or is predicted to exceed the threshold value.

## Description

### Technical field

Examples relate to an apparatus for an optical imaging system, an optical imaging system, such as surgical optical imaging system, a method, and a computer program.

### Background

Light management systems in optical imaging systems, e.g., for neurosurgery, must facilitate work in narrow and deep cavities while avoiding the risk of damaging sensitive tissues. For example, the illuminance is regulated in relation to the working distance, ensuring optimal illumination while protecting sensitive tissues. However, controlling the illuminance based on the working distance may not work for every use case. Thus, there may be a desire for an improved concept for reducing a risk of damaging a tissue.

### Summary

This desire is addressed by the subject-matter of the independent claims.

The concept proposed in the present disclosure is based on the insight, that an action could be triggered if the temperature of a human tissue is exceeded or predicted to exceed a threshold value. The action triggered may allow to reduce a risk of damaging the human tissue and/or to inform the user about the risk of damaging the human tissue. In this way, the likelihood of damage to a human tissue can be reduced and/or can be made dependent on the choice of the user.

Examples provide an apparatus for an optical imaging system comprising one or more processors and one or more storage devices. The apparatus may be configured to receive image data of a human tissue. The apparatus is configured to obtain thermal sensor data of a thermal sensor indicating a temperature of the human tissue. Further, the apparatus is configured to determine, based on the thermal sensor data, whether the temperature of the human tissue has exceeded a threshold value and/or to predict, based on the thermal sensor data, whether the temperature of the human tissue will exceed the threshold value. The apparatus is further configured to trigger an action if the temperature of the human tissue has exceeded or is predicted to exceed the threshold value. Triggering the action may, for example, allow to reduce a heat input into the human tissue and/or to inform the user of potential damage to the human tissue. In this way, damage to the human tissue can be reduced, e.g., to specific use cases. That is, the thermal sensor data may allow to identify a setting of the optical imaging system that can lead to damage to the human tissue. If the human tissue could be damaged, e.g., the threshold value may be exceeded, the apparatus may, for example, allow to reduce the damage to the human tissue by adjusting the setting of the optical imaging system.

In an example, the apparatus may be configured to generate control data for triggering an adjustment of the setting of the optical imaging system if the temperature of the human tissue has exceeded or is predicted to exceed the threshold value. Further, the apparatus may be configured to transmit the control data for adjusting the setting of the optical imaging system for reducing a heat input into the human tissue. Reducing a heat input into the human tissue may allow to reduce a temperature increase of the human tissue caused by the optical imaging system. Reducing the temperature increase may allow to reduce damage to the human tissue. That is, the setting of the optical imaging system can be adjusted, such that the temperature of the human tissue does not exceed or is predicted to not exceed the threshold value. The heat transfer into the human tissue may be a radiative heat transfer caused by an illumination source of the optical imaging system.

In an example, the control data may indicate an adjustment of a setting of an illumination source and/or a working distance of the optical imaging system. Adjusting the setting of the illumination source and/or the working distance may allow to decrease a light intensity incident on the human tissue. In this way, damage to the human tissue can be reduced by reducing a heating of the human tissue caused by an illumination source of the optical imaging system.

In an example, the apparatus may be configured to generate warning data indicating the exceedance or the predicted exceedance of the threshold value if the temperature of the human tissue has exceeded or is predicted to exceed the threshold value. Further, the apparatus may be configured to transmit the warning data for displaying on the display device. The warning data may allow to inform a user about potential and/or actual damage to the human tissue.

Thus, the user can decide whether damage to the human tissue is acceptable or not. For example, the user may need bright illumination during certain surgical process steps performed using the optical imaging system. For these surgical process steps the user can accept or tolerate damage to the human tissue up to a certain level (e.g., 1st degree burn). For other surgical process steps the user may try to avoid any damage to the human tissue. Thus, generating the warning data may allow the user to customize the adjustment of the setting of the optical imaging system.

In an example, the apparatus may be configured to receive user data indicating an action of the user in response to transmitting the warning data and to generate, based on the user data, control data for triggering an adjustment of a setting of the optical imaging system. Further, the apparatus may be configured to transmit the control data for adjusting the setting of the optical imaging system for reducing a heat input into the human tissue. In this way, the setting of the optical imaging system may only be adjusted if the adjustment is approved by the user. For example, if the user does not react to the warning data in a predefined time, the heat input into the human tissue may be reduced as default setting. In this way, the user data can allow the user to customize the setting of the optical imaging system to the needs of the user.

In an example, the apparatus may be configured to generate, based on the control data, feedback data indicating the adjustment of the optical imaging system and to transmit the feedback data for displaying on the display device. Generating the feedback data may allow to inform the user about the adjustment of the optical imaging system. Thus, awareness of the user can be improved. For example, once the setting of the optical imaging system has been adjusted, the user can interrupt a surgical process step for a certain period of time, e.g. to cool down the human tissue, and resume the surgical process step after cooling down and restoring the setting of the optical imaging system.

In an example, the apparatus may be configured to obtain image data of a view of the human tissue from an optical imaging sensor and to determine the threshold value based on the image data. Determining the threshold temperature based on the image data may allow to set the threshold temperature for an actual use case. For example, different human tissues may have different threshold values at which damage occurs. Thus, adjusting the threshold value based on the image data may allow to improve the usability of the optical imaging system and/or to reduce damage to the human tissue.

In an example, the thermal sensor data may indicate a time series of the temperature of the human tissue. Further, the apparatus may be configured to predict whether the threshold value is predicted to be exceeded based on the time series. Using the time series may allow to improve a prediction of the exceedance of the threshold value.

In an example, the threshold value may have a predefined relation to a damage value indicating a temperature at which the human tissue is damaged. That is, the threshold value may relate to a temperature of the human tissue at which the human tissue is damaged. For example, the damage value may be the temperature at which the human tissue sustains thermal damage. Thus, the threshold value may allow to prevent damage to the human tissue-based on an actual value at which damage to the human tissue would occur.

Examples provide an optical imaging system comprising an apparatus as described above.

In an example, the optical imaging system may comprise a thermal sensor. The thermal sensor may be arranged beside an optical path of the optical imaging system. That is, the thermal sensor may not be capable of measuring an image of the human tissue using optical elements of the optical imaging system.

In an example, the optical imaging system may further comprise an optic carrier. The thermal sensor may be arranged on an outside of the optic carrier. That is, the thermal sensor may not be housed by the optic area. For example, the optic carrier may house optical imaging sensors part of the microscope of the optical imaging system. That is, the thermal sensor may not affect image acquisition using the optical imaging system.

Examples provide a method for an optical imaging system. The method may comprise receiving image data of a human tissue and obtaining thermal sensor data of a thermal sensor indicating a temperature of a human tissue. Further, the method comprises determining, based on the thermal sensor data, whether the temperature of the human tissue has exceeded a threshold value and/or predict, based on the thermal sensor data, whether the temperature of the human tissue will exceed the threshold value. The method further comprises triggering an action if the temperature of the human tissue has exceeded or is predicted to exceed the threshold value.

Various examples of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
- Figs. 1a and 1b: show schematic diagrams of an example of an apparatus for an optical imaging system and of a corresponding optical imaging system comprising the apparatus;
- Fig. 2: shows an example of parts of an optical imaging system;
- Fig. 3: shows a flow chart of an example of a method for an optical imaging system; and
- Fig. 4: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

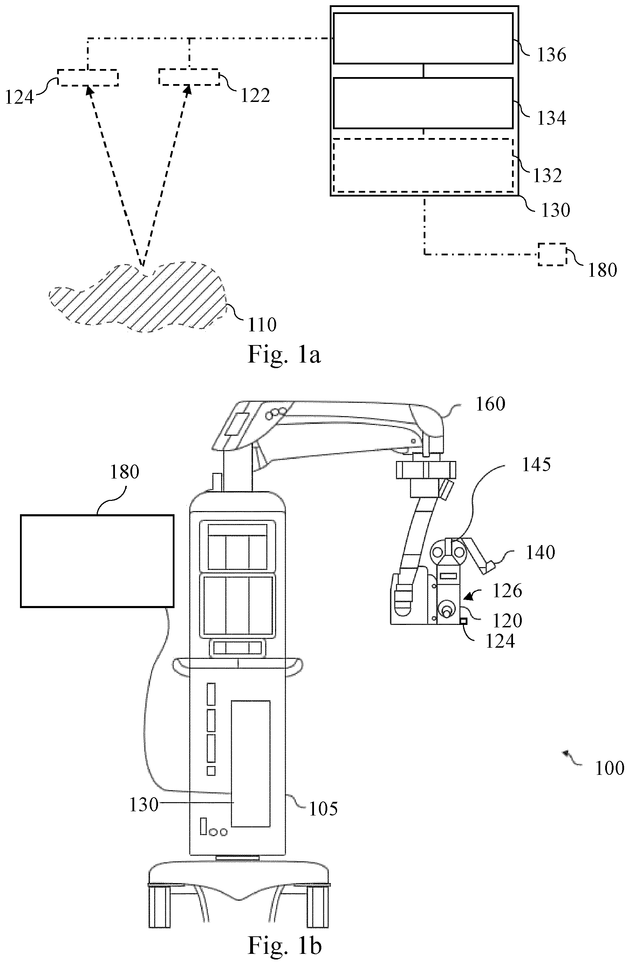

Figs. 1a and 1b show schematic diagrams of an example of an apparatus 130 for an optical imaging system 100 and of a corresponding optical imaging system 100 comprising the apparatus 130. The apparatus 130 is tasked with controlling various aspects of a microscope 120 of the optical imaging system 100, which may be a surgical optical imaging system, and of the entire optical imaging system 100 and/or with processing various types of sensor data of the optical imaging system 100. Consequently, the apparatus 130 may be implemented as a computer system, which interfaces with the various components of the optical imaging system 100, e.g., a thermal sensor 124 and/or an optical imaging sensor 122. The apparatus 130 may be part of the optical imaging system 100. Alternatively, the apparatus 130 may be communicatively coupled to the optical imaging system 100. For example, the apparatus 130 may be a ready-to-use-module, that could be connected to the optical imaging system 100. Alternatively, the apparatus 130 may be part of the thermal sensor 124.

The apparatus 130 comprises, as shown in Fig. 1a, one or more processors 134 and one or more storage devices 136. Optionally, the apparatus 130 further comprises one or more interfaces 132. The one or more processors 134 are coupled to the one or more storage devices 136 and to the optional one or more interfaces 132. In general, the functionality of the apparatus 130 may be provided by the one or more processors 134 (for triggering an action), in conjunction with the one or more interfaces 132 (for exchanging information, e.g., with the thermal sensor 124) and/or with the one or more storage devices 136 (for storing and/or retrieving information).

The apparatus 130 may be configured to receive image data of a human tissue. Receiving the image data of a human tissue 110 is optional. For example, the image data may be received from an optical imaging sensor 122 of the optical imaging system 100. That is, the image data may be acquired using an optical imaging sensor 122 part of the microscope 120 of the optical imaging system 100. In this case, the image data may comprise an enlarged view of the human tissue. Alternatively, the image data may be received from a thermal sensor 124. For example, the image data may be received from the same thermal sensor as the thermal sensor data. In this case, the image data and the thermal sensor data may be measured from the same thermal sensor 124.

Further, the apparatus 130 is configured to obtain thermal sensor data of a thermal sensor indicating a temperature of the human tissue. The thermal sensor data may be obtained by receiving from the thermal sensor 124. Alternatively, the thermal sensor data may be obtained by measuring using the thermal sensor. For example, the apparatus 130 may be part of the thermal sensor 124. Thermal sensor data may refer to information gathered by a thermal sensor configured to measure temperature and/or detect heat levels of a human tissue 110. For example, the thermal sensor 124 may be an infrared sensor. Thus, the thermal sensor data may be data acquired via an infrared sensor. The thermal sensor data may be, for example, a baseline value indicating the ambient and/or specific temperature of the human tissue 110 or may serve as a reference point for measuring deviations in temperature, indicating either the surrounding environment or the temperature of the human tissue 110.

The thermal sensor data can be used by the apparatus 130 to determine a current and/or future temperature of the human tissue 110. Thus, the apparatus 130 can determine potential damage to the human tissue 110 based on the temperature derived from the thermal sensor data. In this way, damage or future damage to the human tissue 100 can be determined in an improved way.

Therefore, the apparatus 130 is configured to determine, based on the thermal sensor data, whether the temperature of the human tissue 110 has exceeded a threshold value and/or to predict, based on the thermal sensor data, whether the temperature of the human tissue 110 will exceed the threshold value.

In an example, the threshold value may have a predefined relation to a damage value indicating a temperature at which the human tissue 110 is damaged. That is, the threshold value may be related to a damage value that makes it possible to determine a damage to the human tissue 110.

For example, the threshold value may be identical to the damage value. In this case an exceedance of the threshold value can indicate a damage to the human tissue 110 and/or a need to protect the human tissue 110. Alternatively, the threshold value may be different from the damage value, e.g., by a predefined temperature difference. In this way, an exceedance of the threshold value may trigger an action to prevent damage to the human tissue 110 before damage occurred. This can be advantageous for sensitive human tissues that could easily be damaged. Further, the prediction of the exceedance of the threshold value may also allow trigger an action to prevent damage to the human tissue 110 before damage occurred. Additionally, the prediction of the exceedance may provide information about time needed to exceed the threshold value. Thus, the prediction of the exceedance may allow to trigger the action such that no damage to the human tissue 110 occurs. In this way, the human tissue 110 can be better protected against damage, e.g., burning.

Further, apparatus 130 is configured to trigger an action if the temperature of the human tissue 110 has exceeded or is predicted to exceed the threshold value. The action can be triggered for reducing the damage to the human tissue 110 and/or for informing the user of the optical imaging system 100 about the damage to the human tissue 100.

Indirectly measuring the temperature of the human tissue 110 based on a working distance and an illumination intensity cannot prevent tissue burns in all cases. It is a finding of the inventors, that burning of the human tissue, such as 1st, 2nd or 3rd degree burn, can be reduced or even avoided based on direct measurement of the temperature of the human tissue 110. The direct measurement of the temperature of the human tissue 100 may be performed using the thermal sensor 124. Thus, the thermal sensor data may help to identify a temperature increase of the human tissue 110, e.g., a surgical region. For example, the thermal sensor data may provide a direct measure of the temperature of the human tissue 110. The direct measure of the temperature of the human tissue 110 can be used to trigger the action to reduce or prevent damage or inform the user about potential damage to the human tissue 110.

There are a variety of different types of optical imaging systems. If the optical imaging system 100 is used in the medical or biological fields, the human tissue 110 may be a sample of organic tissue, e.g., arranged within a petri dish or present in a part of a body of a patient. In some examples of the present disclosure, e.g., as shown in Fig. 1b, the optical imaging system 100 may be a surgical optical imaging system, e.g., an optical imaging system that is to be used during a surgical procedure, such as an oncological surgical procedure or during tumor surgery. However, the proposed concept may also be applied to other types of microscopes, e.g., a microscope in a laboratory or a microscope for the purpose of material inspection.

Fig. 1b shows a schematic diagram of an example of a surgical optical imaging system 100 comprising the microscope 120 and the apparatus 130. In general, a (surgical) optical imaging system is a system that comprises a microscope 120 and additional components, which are operated together with the microscope 120. In other words, a (surgical) optical imaging system is a system that comprises the microscope 120 and one or more additional components, such as the apparatus 130 (which may be a computer system being adapted to control the microscope 120 and, for example, trigger the action), an illumination system (which is used to illuminate a human tissue 110 being imaged by the microscope 120 and can be controlled by the apparatus 130), additional sensors, displays etc.

The optical imaging system 100 shown in Fig. 1b comprises a number of optional components, such as a base unit 105 (which may comprise the apparatus 130) with a stand, ocular displays 140, 145 that are arranged at the microscope 120, a display device 180, and a (robotic or manual) arm 160 which holds the microscope 120 in place, and which is coupled to the base unit 105 and to the microscope 120. In general, these optional and non-optional components may be coupled to the apparatus 130, which may be configured to control and/or interact with the respective components.

In an example, the apparatus 130 may be configured to generate control data for triggering an adjustment of the setting of the optical imaging system 100 if the temperature of the human tissue 110 has exceeded or is predicted to exceed the threshold value. Further, the apparatus 130 may be configured to transmit the control data for adjusting the setting of the optical imaging system 100 for reducing a heat input into the human tissue 110. For example, the adjustment of the setting of the optical imaging system 100 may be for causing a reduction of the temperature of the human tissue 110. Reducing the heat input into the human tissue 110 may allow to decrease the temperature or temperature increase of the human tissue 110. In this way, the probability that the human tissue 110 will be damaged can be reduced.

The transfer into the human tissue 110 may be radiated heat transfer caused by an illumination of the optical imaging system 100, e.g., an illumination source used to illuminate the human tissue 110 for image acquisition using the microscope 120. Thus, use of the optical imaging system 100 may result in burning of the human tissue 110 due to the current setting of the optical imaging system 100. Therefore, adjusting the setting of the optical imaging system 100 may allow to reduce the temperature or temperature increase of the human tissue 110.

In an example, the control data may indicate an adjustment of a setting of an illumination source and/or a working distance of the optical imaging system 100. That is, the heat input into the human tissue 110 can be decreased by adjusting the radiation of the illumination source and/or the working distance. For example, if the temperature of the human tissue 110 has exceeded the threshold value, the intensity of the illumination source can be decreased and/or the working distance can be increased.

The adjustment of the setting of the illumination source, e.g., the intensity and/or the working distance, may depend on the threshold value, a current surgical procedure and/or a user (the setting of the illumination source may be customized). For example, different users may accept or tolerate different levels of damage to the human tissue 110 during operation (e.g., depending on the operation or the surgical process step of the operation), as a reduction in damage may result in reduced usability of the optical imaging system 100. That is, there may be a trade-off between reducing damage to the human tissue 110 and the usability of the optical imaging system 100. For example, an adjustment of the intensity of the illumination source may depend on a user of the optical imaging system 100.

Further, to account for this trade-off the apparatus 130 may be configured to inform the user about the potential damage to the human tissue 110. In an example, the apparatus 130 may be configured to generate warning data indicating the exceedance or the predicted exceedance of the threshold value if the temperature of the human tissue 110 has exceeded or is predicted to exceed the threshold value. Further, the apparatus 130 may be configured to transmit the warning data for displaying on the display device 180. The generation of the warning data may allow to inform the user about a potential damage to the human tissue 110. For example, there may be different degrees of damage such as 1st, 2nd or 3rd degree burn. The user may be prepared to accept a certain amount of damage during the ongoing surgical process step, as the illumination intensity may be required for the surgical process step. Thus, the user can be informed about the potential damage to the human tissue 110 and may decide whether the damage is acceptable or not. Optionally or alternatively, the warning data may indicate a time to the predicted exceedance of the threshold value. In this way, the user can be informed of the time remaining to use the current setting without damage to human tissue 110 occurring. Therefore, the user can adjust the ongoing surgical process step accordingly, e.g., reduce the illumination intensity and optionally interrupt the surgical process step and resume it after the human tissue 110 has cooled down.

In an example, the apparatus 130 may be configured to receive user data indicating an action of the user in response to transmitting the warning data. The action of the user may be a user input using an input device, such like a mouse or keyboard and/or a user utterance, for example. Optionally or alternatively, the action of the user can also consist of no interaction taking place. This means that if there is no interaction, a standard action can be triggered, e.g. reducing the intensity of the illumination.

The action of the user may indicate an intention of the user to adjust the setting of the optical imaging system. For example, if the user wants to prevent damage to the human tissue 110, the user may not react on the warning data such that a default action to prevent damage to the human tissue 110 may be triggered by the apparatus 130. For example, if the user wishes to use the current setting of the optical imaging system 100 for a longer period of time, they can refuse to adjust the setting. In this way, the adjustment of the setting of the optical imaging system 100 can be customized which may improve user experience. Therefore, the apparatus 130 may be further configured to generate, based on the user data, control data for triggering an adjustment of a setting of the optical imaging system 100. Further, the apparatus 130 may be configured to transmit the control data for adjusting the setting of the optical imaging system 100 for reducing a heat input into the human tissue 110.

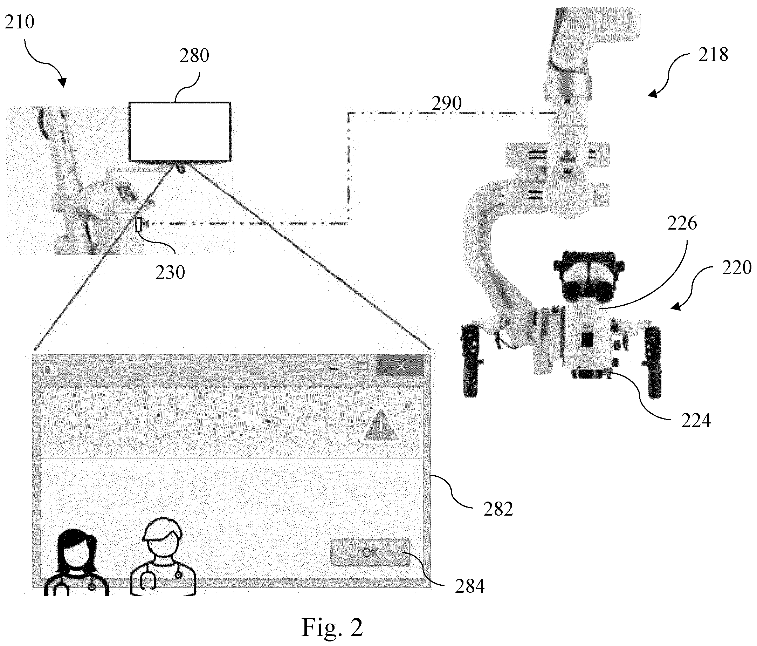

In an example, the apparatus 130 may be configured to generate, based on the control data, feedback data indicating the adjustment of the optical imaging system 100 and to transmit the feedback data for displaying on the display device 180 (see also Fig. 2). Generating the feedback data may allow to inform the user about the adjustment of the optical imaging system 100. For example, the user can be informed about a reduction of the illumination intensity of the illumination source of the optical imaging system 100.

In an example, the apparatus 130 may be configured to obtain image data of a view of the human tissue 110 from an optical imaging sensor 122 and to determine the threshold value based on the image data. The threshold value may depend on the human tissue 110. For example, thermal damage varies among human tissues due to differences in water content, vascularization, pigmentation, and density, all of which affect how each tissue absorbs, scatters, and dissipates light. Thus, the damage value and therefore the threshold value may vary depending on the characteristic of the human tissue 110. The image data may allow to determine the threshold value (e.g., by determining the damage value) for the human tissue 110. For example, image recognition may be used to determine a structure of the human tissue 110. Based on the determined structure the threshold value may be determined, e.g., loaded from a database. In this way, the threshold value can be adapted during an ongoing surgical process, e.g., the threshold value may be different for different surgical process steps. This may improve a usability of the optical imaging system 100.

In an example, the thermal sensor data may indicate a time series of the temperature of the human tissue 110. Further, the apparatus 130 may be configured to predict whether the threshold value is predicted to be exceeded based on the time series. The time series of the temperature of the human tissue 110 can be analyzed using mathematical algorithm to predict the temperature of the human tissue 110 in the future, for example. For example, the prediction may be based on an extrapolation of the temperature of the human tissue 110.

In an example, the optical imaging system 100 may comprise a thermal sensor 124. The thermal sensor 124 may be arranged beside an optical path of the optical imaging system 100. For example, the thermal sensor 124 may not be used to acquire a magnified view of the human tissue 110. The thermal sensor 124 can only be used to measure the thermal sensor data.

In an example, the optical imaging system 100 may further comprise an optic carrier 126. The thermal sensor 124 may be arranged on an outside of the optic carrier 126. The optic carrier 126 may house the microscope 120 or parts of the microscope, e.g., lenses, optical imaging sensors, a diaphragm. That is, the optic carrier 126 can be a housing of the microscope 120. Arranging the thermal sensor 124 on an outside of the optic carrier 126 may allow to facilitate the arrangement. For example, the thermal sensor 124 may be a ready-to-use-module which could be attached to the optical imaging system 100. Thus, the thermal sensor 124 can be retrofitted. In this way, an effect of the thermal sensor 124 on the performance of the optical imaging system 100 can be reduced or even avoided. Further, a low-cost set up can be provided.

As shown in Fig. 1a the optional one or more interfaces 132 is coupled to the respective one or more processors 134 at the apparatus 130. In examples the one or more processors 134 may be implemented using one or more processing units, one or more processing devices, any means for processing, such as a processor, a computer or a programmable hardware component being operable with accordingly adapted software. Similar, the described functions of the one or more processors 134 may as well be implemented in software, which is then executed on one or more programmable hardware components. Such hardware components may comprise a general-purpose processor, a Digital Signal Processor (DSP), a micro-controller, etc. The one or more processors 134 is capable of controlling the one or more interfaces 132, so that any data transfer that occurs over the one or more interfaces 132 and/or any interaction in which the one or more interfaces 132 may be involved may be controlled by the one or more processors 134.

In an embodiment the apparatus 130 may comprise a memory, e.g., the one or more storage devices 136 and at least one or more processors 134 operably coupled to the memory and configured to perform the method described below.

In examples the one or more interfaces 132 may correspond to any means for obtaining, receiving, transmitting or providing analog or digital signals or information, e.g. any connector, contact, pin, register, input port, output port, conductor, lane, etc. which allows providing or obtaining a signal or information. The one or more interfaces 132 may be wireless or wireline and it may be configured to communicate, e.g., transmit or receive signals, information with further internal or external components.

The apparatus 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

More details and aspects are mentioned in connection with the examples described below. The example shown in Fig. 1 may comprise one or more optional or additional features corresponding to one or more aspects mentioned in connection with the proposed concept or one or more examples described below (e.g., Fig. 2 - 4).

Fig. 2 shows an example of parts of an optical imaging system. The optical imaging system may comprise a base component 210 and an arm component 218. The base component 210 may comprise a display device 280 and an apparatus 230. The apparatus 230 can be an apparatus as described with reference to Fig. 1. The arm component 218 may comprise the microscope 220. The microscope 220 may comprise an optic carrier 226 housing the optical components of the microscope 220. Further, the arm component 218 may comprise a thermal sensor 224. The thermal sensor 224 may be attached to an outside of the optic carrier 226. That is, the thermal sensor 224 may be arranged beside an optical path of the microscope 220.

The apparatus 230 may receive thermal sensor data from the thermal sensor 224 (indicated by 290). The apparatus 230 may determine based on the thermal sensor data whether a temperature of a human tissue has exceeded or will exceed a threshold value. If the temperature of the human tissue has exceeded or will exceed the threshold value, the apparatus 230 trigger an action. For example, as shown in Fig. 2, the action may comprise adjusting the setting of the optical imaging system and generating feedback data for informing the user. As can be seen from the graphical user interface 282 displayed on the display device 280 in Fig. 2, the setting of the optical imaging system may be adjusted to reduce an intensity of the illumination source of the optical imaging system. For example, the intensity can be reduced from 100% to 70% if the human tissue temperature has reached the 1st degree burn. That is, the graphical user interface 282 can be a warning dialog for informing the user about damage to the human tissue. For example, the graphical user interface 282 may be a tissue burn warning. The graphical user interface 282 may provide an icon 284 four allowing the user to provide feedback. For example, the icon 284 can be selected by the user to accept the proposed adjustment of the optical imaging system. Thus, the user can use the icon 284 to provide feedback data for adjusting the setting of the optical imaging system.

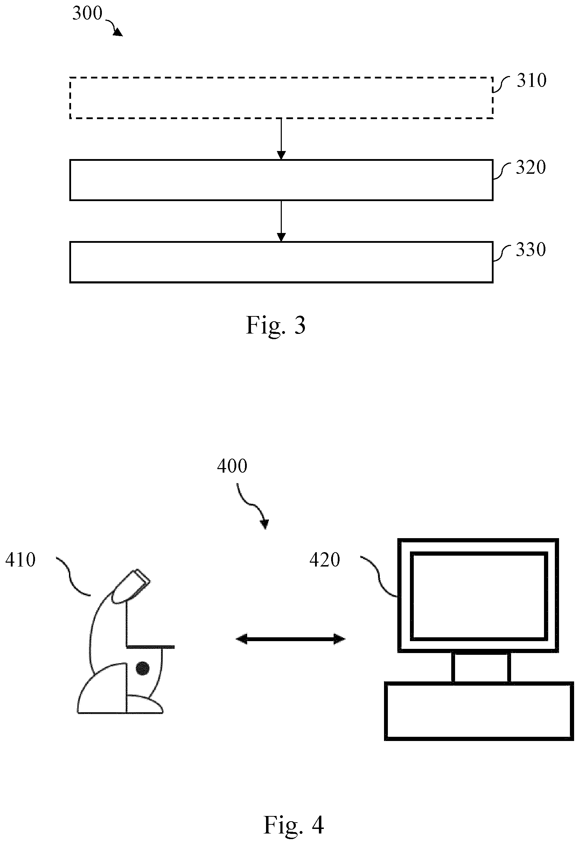

More details and aspects are mentioned in connection with the examples described above and/or below. The example shown in Fig. 2 may comprise one or more optional or additional features corresponding to one or more aspects mentioned in connection with the proposed concept or one or more examples described above (e.g., Fig. 1) and/or below (e.g., Fig. 3 - 4).

Fig. 3 shows a flow chart of an example of a method 300 for an optical imaging system. The method may comprise receiving 310 image data of a human tissue and obtaining thermal sensor data of a thermal sensor indicating a temperature of a human tissue. Further, the method 300 comprises determining 320, based on the thermal sensor data, whether the temperature of the human tissue has exceeded a threshold value and/or predict, based on the thermal sensor data, whether the temperature of the human tissue will exceed the threshold value. The method 300 further comprises triggering 320 an action if the temperature of the human tissue has exceeded or is predicted to exceed the threshold value. The method 300 may be performed or processed by an apparatus as described above, e.g., with reference to Fig. 1 and/or Fig. 2.

More details and aspects are mentioned in connection with the examples described above and/or below. The example shown in Fig. 3 may comprise one or more optional or additional features corresponding to one or more aspects mentioned in connection with the proposed concept or one or more examples described above (e.g., Fig. 1 - 2) and/or below (e.g., Fig. 4).

Some embodiments relate to a microscope comprising an apparatus as described in connection with one or more of the Figs. 1 to 2. Alternatively, a microscope or an optical imaging system can be communicatively connected to an apparatus as described in connection with one or more of the Figs. 1 to 2. Fig. 4 shows a schematic illustration of a system 400, e.g., an optical imaging system, configured to perform a method described herein, e.g., with reference to Fig. 3. The system 400 comprises a microscope 410 and a computer system 420. The microscope may comprise the apparatus as described above, e.g., with reference to Fig. 1 or Fig. 2. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

More details and aspects are mentioned in connection with the examples described above. The example shown in Fig. 4 may comprise one or more optional or additional features corresponding to one or more aspects mentioned in connection with the proposed concept or one or more examples described above (e.g., Fig. 1 - 3).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

If some aspects have been described in relation to a device or system, these aspects should also be understood as a description of the corresponding method and vice versa. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 optical imaging system
105 base
110 human tissue
120 microscope
122 optical imaging sensor
124 thermal sensor
126 optic carrier
130 apparatus
132 interface
134 processor
136 storage device
140, 145 ocular display
160 arm
180 head-mounted display
210 base component
218 arm component
220 microscope
224 thermal sensor
230 apparatus
280 display device
282 graphical user interface
284 icon
400 system
410 microscope
420 computer system

## Claims

1. An apparatus (130) for an optical imaging system, comprising one or more processors (134) and one or more storage devices (136), wherein the apparatus (130) is configured to:
receive image data of a human tissue;
obtain thermal sensor data of a thermal sensor indicating a temperature of the human tissue;
at least one of
determine, based on the thermal sensor data, whether the temperature of the human tissue has exceeded a threshold value or
predict, based on the thermal sensor data, whether the temperature of the human tissue will exceed the threshold value; and
trigger an action if the temperature of the human tissue has exceeded or is predicted to exceed the threshold value.

2. The apparatus according to claim 1, wherein the apparatus (130) is configured to:
generate control data for triggering an adjustment of a setting of the optical imaging system if the temperature of the human tissue has exceeded or is predicted to exceed the threshold value; and
transmit the control data for adjusting the setting of the optical imaging system for reducing a heat input into the human tissue.

3. The apparatus according to claim 2, wherein
the control data indicates an adjustment of at least one of a setting of an illumination source or a working distance of the optical imaging system.

4. The apparatus according to any one of the preceding claims, wherein the apparatus is configured to:
generate warning data indicating the exceedance or the predicted exceedance of the threshold value if the temperature of the human tissue has exceeded or is predicted to exceed the threshold value; and
transmit the warning data for displaying on a display device.

5. The apparatus according to claim 4, wherein the apparatus is configured to:
receive user data indicating an action of the user in response to transmitting the warning data;
generate, based on the user data, control data for triggering an adjustment of a setting of the optical imaging system; and
transmit the control data for adjusting the setting of the optical imaging system for reducing a heat input into the human tissue.

6. The apparatus according to any one of the claims 2-5, wherein the apparatus is configured to:
generate, based on the control data, feedback data indicating the adjustment of the optical imaging system; and
transmit the feedback data for displaying on a display device.

7. The apparatus according to any one of the preceding claims, wherein the apparatus is configured to:
obtain image data of a view of the human tissue from on optical imaging sensor; and
determine the threshold value based on the image data.

8. The apparatus according to any one of the preceding claims, wherein the apparatus is configured to:
obtain state data indicating a state of a workflow performed using the optical imaging system; and
determine the threshold value based on the state data.

9. The apparatus according to any one of the preceding claims, wherein
the thermal data indicate a time series of the temperature of the human tissue, and wherein the apparatus is configured to predict whether the threshold value is predicted to be exceed based on the time series.

10. The apparatus according to any one of the preceding claims,
wherein the threshold value has a predefined relation to a damage value indicating a temperature at which the human tissue is damaged.

11. An optical imaging system (100), comprising
an apparatus (130) according to any one of the preceding claims.

12. The optical imaging system according to claim 11, further comprising
a thermal sensor, wherein the thermal sensor is arranged beside an optical path of the optical imaging system.

13. The optical imaging system according to claim 12, further comprising
an optic carrier, wherein the thermal sensor is arranged on an outside of the optic carrier.

14. A method for an optical imaging system, comprising:
receiving image data of a human tissue;
obtaining thermal sensor data of a thermal sensor indicating a temperature of a human tissue;
at least one of
determining, based on the thermal sensor data, whether the temperature of the human tissue has exceeded a threshold value or
predict, based on the thermal sensor data, whether the temperature of the human tissue will exceed the threshold value; and
triggering an action if the temperature of the human tissue has exceeded or is predicted to exceed the threshold value.

15. A computer program with a program code for performing the method according to claim 14 when the computer program is executed on a processor.
